# EUROPEAN PATENT SPECIFICATION

(11) **EP 0 398 660 B1**
(45) Date of publication and mention of the grant of the patent: **10.08.1994**
(21) Application number: 90305241.3
(22) Date of filing: 15.05.1990
(51) Int. Cl.: A61B 5/0402, A61B 5/0404

(54) **Apparatus for recording an electrocardiogram**
Aufzeichnungsgerät eines Elektrokardiogramms
Appareil d'enregistrement d'un électrocardiogramme

(30) Priority: 16.05.1989 JP 123563/89
(43) Date of publication of application: 22.11.1990
(73) Proprietor: SHARP KABUSHIKI KAISHA, Osaka 545 (JP)
(72) Inventor: Wada, Yoshihiro, Nara-shi, Nara-ken (JP)
(74) Representative: Brown, Kenneth Richard

(56) References cited:
- EP-A- 0 170 448
- EP-A- 0 265 694
- WO-A-87/06447
- WO-A-88/05282

## Description

The present invention relates to a portable recording apparatus for an electrocardiogram. More particularly it relates to such a recording apparatus having a display unit such as a liquid crystal display device for displaying recorded electrocardiographic waves such as Holter electrocardiograph.

WO 87/06447 discloses an electrocardiograph as set out in the preamble of claim 1, which converts input analog electrocardiographic electrical signals into digital form, stores the digital signals and graphically displays the digital signals on an LCD screen.

The inventors of the present application know of another electrocardiogram recording apparatus capable of confirming the recording status and making diagnoses easily as irregular pulses by displaying the recorded electrocardiographic waves on a liquid crystal display device.

In this apparatus, the electrocardiographic wave is sequentially displayed as a static wave form from left to right on the display screen. Once the wave reaches the right end of the display screen, a new wave is sequentially displayed by erasing the previous waveform, which has just been displayed. This method of displaying the waveform has been commonly used for monitoring the electrocardiogram.

Such a known apparatus as described above has disadvantages. For example, when three QRS groups (spikes corresponding to the start of a ventricular systole), indicating the time change of an electric potential with heart pulsations, are displayed from left to right on the display screen in order, the display position of the QRS group displayed on the leftmost end of the screen is not fixed, but is changed when displaying a new waveform thereon. As a result, it is difficult to visualize intervals between the R wave in a QRS group and the R wave in the next QRS group to be displayed to the right of the already generated QRS group, that is, it is difficult to visualize a change of R-R intervals (the change of a heart-beat rate). Furthermore it is difficult to capture the R-R interval between the R wave in the QRS group on the rightmost end of the display screen and the R wave in the next QRS group to be displayed on the leftmost end of the screen, since the display between the R-R interval will be interrupted.

The present invention aims to alleviate the above-described disadvantages in prior art electrocardiographs.

According to the invention, there is provided an apparatus for recording an electrocardiogram, said apparatus having a key input unit, an electrocardiogram measuring circuit for converting the electrocardiogram into digital data and for outputting said digital data, a memory means for storing digital data outputted from the electrocardiogram measuring circuit, and a display unit for displaying the electrocardiogram waveforms on a display screen thereof, characterized in that
said memory means is arranged to store the digital data defining the electrocardiogram waveform occurring during a predetermined time period; and in that the apparatus further comprises
display control means for controlling the display unit in accordance with the stored digital data to display a portion of the stored electrocardiograph waveform, comprising a plurality of successive QRS groups along a time axis such that the displayed electrocardiogram waveform portion can be shifted along said time axis, and such that a predetermined reference point of the leftmost one of the displayed QRS groups is located at a predetermined position along said time axis.

Preferably, the apparatus further includes means for calculating heart-beat rates instantaneously by using the inverse of the time difference between the reference points of adjacent QRS groups.

More preferably, the apparatus further includes a heart-beat rate memory for storing the instantaneous heart-beat rates calculated by the calculating means.

The predetermined time period for the memory unit is preferably ten minutes.

Furthermore, the apparatus preferably includes detecting means adapted to detect, for each QRS group, a negative maximum point exceeding a predetermined threshold value by differentiating the electrocardiographic wave stored in the memory means.

The negative maximum points detected by the detecting means are preferably taken as the reference points for the respective QRS groups.

Alternatively, the reference point in each QRS group may be at the position of a peak of one spike in said QRS group. Such one spike may be an R wave of the QRS group.

Preferably, the display control means controls the display means to display three QRS groups simultaneously, with the reference point of the QRS group which is leftmost among the three displayed QRS groups located at said predetermined position.

The display unit also may display the instantaneous heart-beat rates.

The display unit is preferably a liquid crystal display device.

The display control means is preferably operable to cause the displayed electrocardiogram waveform to shift along the time axis by an amount corresponding to a predetermined number of QRS groups, said number preferably being variable in accordance with operation of said key input unit by the user.

A preferred embodiment of the present invention will now be described by way of example with reference to the accompanying drawings, in which:
Fig. 1 shows a block diagram illustrating of an embodiment the electrocardiogram recording apparatus according to the present invention;
Fig. 2 shows an electrocardiogram and one example of the display screen in accordance with the electrocardiogram recording apparatus shown in Fig. 1;
Fig. 3 shows waveforms for explaining a detection of the reference point according to the electrocardiogram recording apparatus of Fig. 1;
Fig. 4 illustrates the operation keys of the electrocardiogram recording apparatus shown in Fig. 1;
Fig. 5 shows an electrocardiogram and the display screen when the display screen of the electrocardiogram shown in Fig. 2 is shifted rightward; and
Fig. 6 shows the electrocardiogram and the display screen when the display screen shown in Fig. 5 is further shifted rightward.

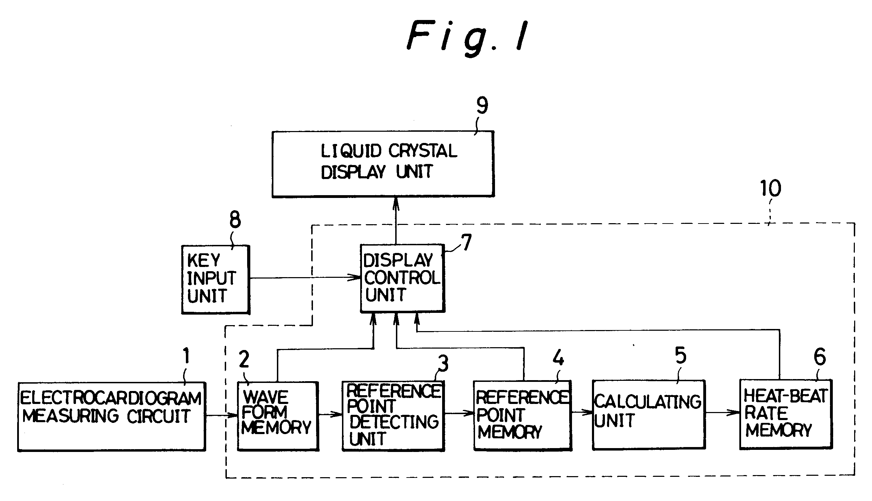

As shown in Fig. 1, numeral 1 indicates an electrocardiogram measuring circuit for measuring an electrocardiogram, for converting the electrocardiogram into digital data, and for outputting digital data. Numeral 2 indicates a waveform memory for storing the electrocardiographic wave data of the digital data outputted from the electrocardiogram measuring circuit 1 for a fixed time, for example, ten minutes. Numeral 3 denotes a reference point detecting unit for detecting negative maximum points exceeding the fixed threshold values to set the detected negative maximum points to reference points in QRS groups by differentiating the electrocardiographic wave data outputted from the waveform memory as will be described later. Numeral 4 indicates a reference point memory for storing the detected reference points outputted from the reference point detecting unit 3, while numeral 5 indicates a calculating unit for calculating instantaneous heart-beat rates by using the inverse of the time difference between the adjacent reference points. Numeral 6 denotes a heart-beat rate memory for storing the instantaneous heart-beat rate calculated by the calculating unit. Numeral 7 indicates a display control unit for controlling, the display of the electrocardiographic wave data after reading out from the waveform memory 2 in response to the key operation of a key input unit 8. Numeral 9 denotes a liquid crystal display unit used as display unit for displaying the electrocardiogram waveforms on the display screen.

In the above-described construction, a microcomputer 10 includes basically the waveform memory 2, the reference point detecting unit 3, the reference point memory 4, the calculating unit 5 for instantaneously calculating heart-beat rate, the heart-beat rate memory 6 and the display control unit 7.

The electrocardiogram recording apparatus according to this embodiment is constructed in the following way so as to easily visualize a change in the R-R intervals and the like.

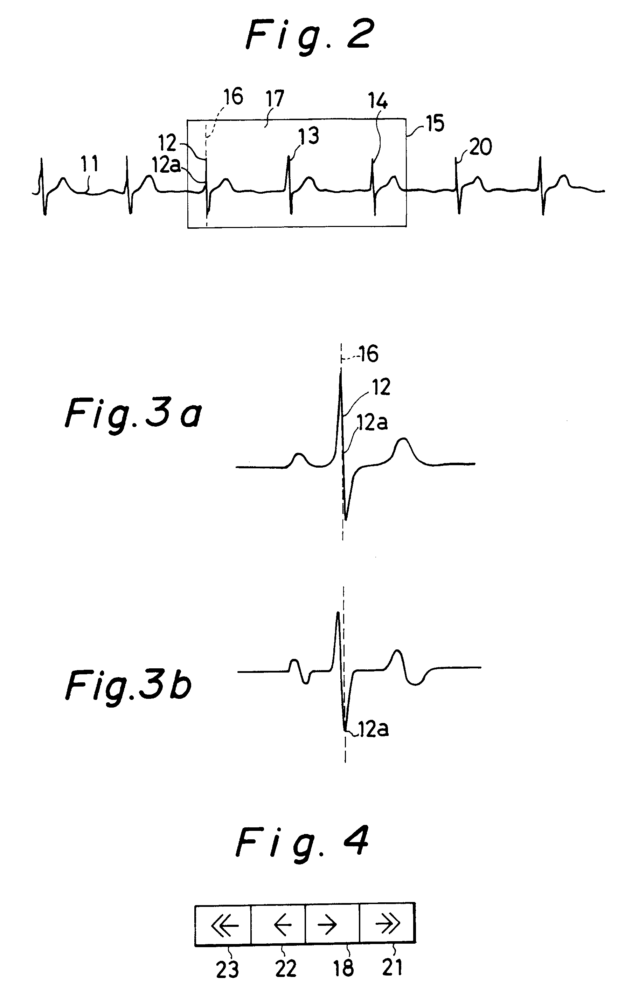

Fig. 2 is a chart showing an electrocardiogram and one example of the display screen in accordance with this invention.

As shown in Fig. 2, three QRS groups 12 to 14 of a continuous electrocardiogram 11 are displayed on a rectangular screen 15. The reference point 12a of the QRS group 12, which is the leftmost among the three QRS groups 12 to 14, is indicated by a broken line 16 positioned at a fixed place on the screen 15. This reference point 12a is a point detected by the reference point detecting unit 3. The reference point 12a is a negative maximum point which exceeds the fixed threshold value of the waveform shown in Fig. 3b. The waveform shown in Fig. 3b is a waveform obtained by differentiating the QRS group 12 of the electrocardiogram waveform shown in Fig. 3a. In other words, the reference point 12a is a sharp point of the change in the electrocardiogram. The reference point detecting unit 3 detects each reference point for each QRS group of the electrocardiogram and stores each reference point in the reference point memory 4.

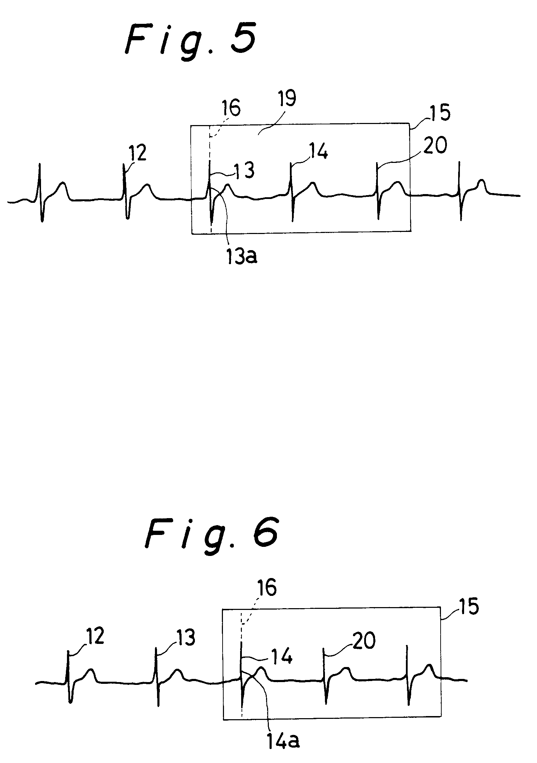

An instantaneous heart-beat rate 17 between the QRS group 12 and the succeeding QRS group 13 is also displayed on the screen 15. Under this display condition, pressing of a one-heart-beat rate stroke key 18 (shown in Fig. 4) of the key input section 8 makes one of the QRS groups shift leftward, that is, pressing the one-heart-beat rate stroke key 18 makes one of the QRS groups shift leftward a distance corresponding to one group QRS of the electrocardiogram. As shown in Fig. 5, the reference point 13a of the QRS group 13 is then displayed at the fixed position 16 on the screen 15, as an instantaneous heart-beat rate 19 between the QRS group 13 and the succeeding QRS group 14 is also displayed, and a succeeding new QRS group 20 is displayed on the screen 15.

Furthermore, under the display condition as shown in Fig. 2, pressing of a screen scroll key 21 (shown in Fig. 4) of the key input section 8, causes the electrocardiogram to shift leftward by a distance corresponding to two QRS groups as shown in Fig. 6. The reference point 14a of the QRS group 14 at the right end of the screen 15 is then displayed at the fixed position 16 on the screen 15.

Upon pressing of a one-heart-beat rate stroke key 22 or a screen scroll key 23 (shown in Fig. 4) of the key input section 8, the electrocardiogram is shifted in a reversed direction of the above-mentioned direction, that is, the electrocardiogram is shifted rightward. Further, when the keys 18, 21, 22 and 23 of the input section 8 are pressed in succession, the screen 15 keeps scrolling with the QRS groups displayed at a rate of three QRS groups per second.

As described above, when three QRS groups are displayed on the screen 15 after the electrocardiogram from the waveform memory 2 has been read, accordingly the display control unit 7 displays the reference point of the QRS group which is the leftmost among such three QRS groups at the fixed position 16 on the screen 15 based on the reference point data from the reference point memory 4. The display control unit 7 also shifts the QRS groups rightward or leftward by a distance corresponding to one or two QRS groups in order to display the QRS groups in response to a key operation of the four keys 18, 21, 22 and 23.

The reference point, displayed on the screen of the liquid crystal display 9, of the QRS group which is the leftmost among the three QRS groups of the electrocardiogram is always displayed accordingly at the predetermined position 16 on the screen 15, and the reference point is shifted either rightward or leftward by a predetermined operation for only predetermined numbers of QRS groups. For the above reasons, it becomes easy to visualize the changes of the intervals between the QRS group at the predetermined position and the succeeding QRS groups, that is, the changes in the R-R intervals. In addition, there is no interruption between the display of the rightmost QRS group and the QRS group which is to be displayed next.

Moreover, since an instantaneous heart-beat rate is also displayed, it is possible to determine a change in the R-R intervals more accurately in this embodiment.

Although the three QRS groups are displayed on the screen 15 in this embodiment, the number of the QRS groups is not limited to three in the present invention. The reference point of the QRS group is not limited to a negative maximum point of the differential waveform, but a R wave, which is a peak wave of the QRS group, may also be utilized.

## Claims

1. An apparatus for recording an electrocardiogram, said apparatus having a key input unit (8), an electrocardiogram measuring circuit (1) for converting the electrocardiogram into digital data and for outputting said digital data, a memory means (2) for storing digital data outputted from the electrocardiogram measuring circuit (1) and defining the electrocardiogram waveform occurring during a predetermined time period, a display unit (9) for displaying the electrocardiogram waveforms on a display screen thereof, and a display control means (7) for controlling the display unit (9) in accordance with the stored digital data to display a portion of the stored electrocardiogram waveform, comprising a plurality of successive QRS groups along a time axis such that the displayed electrocardiogram waveform portion can be shifted along said time axis, characterized in that
said display control means (7) control said display unit (9) such that a predetermined reference point of the leftmost one of the displayed QRS groups is located at a predetermined position along said time axis.

2. An apparatus for recording an electrocardiogram according to claim 1, wherein said apparatus further comprises means (5) for calculating heart-beat rates instantaneously by using the inverse of the time difference between the said reference points of adjacent QRS groups.

3. An apparatus for recording an electrocardiogram according to claim 2, wherein said apparatus further comprises a heart-beat rate memory (8) for storing said instantaneous heart-beat rates calculated by said calculating means.

4. An apparatus for recording an electrocardiogram according to any preceding claim, wherein said predetermined time period is ten minutes.

5. An apparatus for recording an electrocardiogram according to any preceding claim, wherein said apparatus further comprises detecting means (3) which is adapted to detect for each QRS group a negative maximum point exceeding a predetermined threshold value by differentiating said electrocardiographic wave stored in said memory means (2).

6. An apparatus for recording an electrocardiogram according to claim 5, wherein the negative maximum points detected by said detecting means (3) are taken as said reference points for the respective QRS groups.

7. An apparatus for recording an electrocardiogram according to any of claims 1 to 4, wherein said reference point in each QRS group is at the position of a peak of one spike in said QRS group.

8. An apparatus for recording an electrocardiogram according to claim 7, wherein said one spike is an R wave of the QRS group.

9. An apparatus for recording an electrocardiogram according to any preceding claim, wherein said display control means (7) is adapted to control the display unit (9) to display three QRS groups simultaneously, with the reference point of the QRS group which is leftmost among said three displayed QRS groups located at said predetermined position.

10. An apparatus for recording an electrocardiogram according to claim 2 or any claim dependant thereon, wherein said display control means (7) is also adapted to control the display unit (9) for displaying said instantaneous heart-beat rates.

11. An apparatus for recording an electrocardiogram according to any preceding claim, wherein the display unit (9) comprises a liquid crystal display device.

12. An apparatus according to any preceding claim wherein said control means is operable to cause the displayed electrocardiogram waveform to shift along said time axis by an amount corresponding to a predetermined number of QRS groups.

13. An apparatus according to claim 12 wherein said predetermined number is variable in accordance with operation of said key input unit by the user.

## Patentansprüche

1. Gerät zum Aufzeichnen eines Elektrokardiogramms, welches Gerät eine Tasteneingabeeinheit (8), eine Elektrokardiogramm-Meßschaltung (1) zum Umwandeln des Elektrokardiogramms in digitale Daten und zum Ausgeben der digitalen Daten, eine Speichereinrichtung (2) zum Abspeichern der von der Elektrokardiogramm-Meßschaltung (1) ausgegebenen digitalen Daten, die den während einer vorgegebenen Zeitspanne auftretenden Elektrokardiogramm-Meßsignalverlauf festlegen, eine Anzeigeeinheit (9) zum Anzeigen der Elektrokardiogramm-Signalverläufe auf einem Anzeigeschirm, und eine Anzeigesteuereinheit (7) aufweist, um die Anzeigeeinheit (9) abhängig von den abgespeicherten digitalen Daten so zu steuern, daß ein Teil des abgespeicherten Elektrokardiogramm-Signalverlaufs mit mehreren aufeinanderfolgenden QRS-Gruppen entlang der Zeitachse auf solche Weise dargestellt wird, daß der Teil des dargestellten Elektrokardiogramm-Signalverlaufs entlang der Zeitachse verschoben werden kann;
**dadurch gekennzeichnet, daß**
- die Anzeigesteuereinheit (7) die Anzeigeeinheit (9) so steuert, daß ein vorgegebener Bezugspunkt der ganz linken der dargestellten QRS-Gruppen an einer vorgegebenen Position entlang der Zeitachse liegt.

2. Gerät zum Aufzeichnen eines Elektrokardiogramms gemäß Anspruch 1, das ferner eine Einrichtung (5) zum momentanen Berechnen der Herzschlagzahl unter Verwendung des Kehrwertes der Zeitdifferenz zwischen den Bezugspunkten benachbarter QRS-Gruppen aufweist.

3. Gerät zum Aufzeichnen eines Elektrokardiogramms gemäß Anspruch 2, das ferner einen Herzschlagzahlspeicher (8) aufweist, um die durch die Berechnungseinrichtung berechneten momentanen Herzschlagzahlen abzuspeichern.

4. Gerät zum Aufzeichnen eines Elektrokardiogramms nach einem der vorstehenden Ansprüche, bei dem die vorgegebene Zeitperiode zehn Minuten beträgt.

5. Gerät zum Aufzeichnen eines Elektrokardiogramms nach einem der vorstehenden Ansprüche, das ferner eine Erfassungseinrichtung (13) aufweist, die so ausgebildet ist, daß sie für jede QRS-Gruppe das einen vorgegebenen Schwellenwert überschreitende negative Maximum dadurch berechnet, daß sie das in der Speichereinrichtung (2) abgespeicherte Elektrokardiographiesignal differenziert.

6. Gerät zum Aufzeichnen eines Elektrokardiogramms gemäß Anspruch 5, bei dem die von der Erfassungseinrichtung (3) erfaßten Maximalpunkte als die genannten Bezugspunkte für die jeweiligen QRS-Gruppen verwendet werden.

7. Gerät zum Aufzeichnen eines Elektrokardiogramms nach einem der Ansprüche 1 bis 4, bei dem der Bezugspunkt in jeder QRS-Gruppe an der Position des Spitzenwerts eines Spikes in der QRS-Gruppe liegt.

8. Gerät zum Aufzeichnen eines Elektrokardiogramms gemäß Anspruch 7, bei dem der eine Spike das R-Signal der QRS-Gruppe ist.

9. Gerät zum Aufzeichnen eines Elektrokardiogramms nach einem der vorstehenden Ansprüche, bei dem die Anzeigesteuereinrichtung (7) so ausgebildet ist, daß sie die Anzeigeeinheit (9) so steuert, daß sie gleichzeitig drei QRS-Gruppen anzeigt, wobei der Bezugspunkt der ganz linken unter den drei dargestellten QRS-Gruppen an der vorgegebenen Position liegt.

10. Gerät zum Aufzeichnen eines Elektrokardiogramms gemäß Anspruch 2 oder nach einem der von diesem abhängigen Ansprüche, bei dem die Anzeigesteuereinrichtung (7) auch so ausgebildet ist, daß sie die Anzeigeeinheit (9) zum Darstellen der momentanen Herzschlagzahl ansteuert.

11. Gerät zum Aufzeichnen eines Elektrokardiogramms nach einem der vorstehenden Ansprüche, bei dem die Anzeigeeinheit (9) eine Flüssigkristall-Anzeigevorrichtung aufweist.

12. Gerät nach einem der vorstehenden Ansprüche, bei dem die Steuereinrichtung so betrieben werden kann, daß sie dafür sorgt, daß der dargestellte Elektrokardiogramm-Signalverlauf entlang der Zeitachse um ein Stück verschoben wird, das einer vorgegebenen Anzahl von QRS-Gruppen entspricht.

13. Gerät nach Anspruch 12, bei dem die vorgegebene Anzahl abhängig von der Betätigung der Tasteneingabeeinheit durch den Benutzer veränderbar ist.

## Revendications

1. Appareil pour enregistrer un électrocardiogramme, ledit appareil ayant une unité d'entrée à touches (8), un circuit de mesure d'électrocardiogramme (1) pour convertir l'électrocardiogramme en des données numériques et pour délivrer en sortie lesdites données numériques, un moyen de mémoire (2) pour mémoriser des données numériques délivrées en sortie par le circuit de mesure d'électrocardiogramme (1) et définissant la forme d'onde d'électrocardiogramme survenant pendant une période de temps prédéterminée, une unité de visualisation (9) pour afficher les formes d'ondes d'électrocardiogramme sur un écran de visualisation de l'unité de visualisation, et un moyen de commande de visualisation (7) pour commander l'unité de visualisation (9) en accord avec des données numériques mémorisées afin d'afficher une partie de la forme d'onde d'électrocardiographie mémorisée, comprenant une pluralité de groupes QRS successifs le long d'un axe de temps de telle façon que la partie de forme d'onde d'électrocardiogramme affichée peut être décalée le long dudit axe de temps, caractérisé en ce que
ledit moyen de commande de visualisation (7) commande ladite unité de visualisation (9) de telle façon qu'un point de référence prédéterminé du groupe QRS le plus à gauche des groupes QRS affichés est situé à une position prédéterminée le long dudit axe de temps.

2. Appareil pour enregistrer un électrocardiogramme selon la revendication 1, dans lequel ledit appareil comprend également des moyens (5) pour calculer instantanément des fréquences de battement cardiaque en utilisant l'inverse de la différence de temps entre lesdits points de référence de groupes QRS adjacents.

3. Appareil pour enregistrer un électrocardiogramme selon la revendication 2, dans lequel ledit appareil comprend également une mémoire de fréquence de battement cardiaque (8) pour mémoriser lesdites fréquences instantanées de battement cardiaque calculées par lesdits moyens de calcul.

4. Appareil pour enregistrer un électrocardiogramme selon l'une quelconque des revendications précédentes, dans lequel la période de temps prédéterminée est de dix minutes.

5. Appareil pour enregistrer un électrocardiogramme selon l'une quelconque des revendications précédentes, dans lequel ledit appareil comprend également des moyens de détection (3) qui sont adaptés pour détecter pour chaque groupe QRS un point maximum négatif dépassant une valeur de seuil prédéterminée en différentiant ladite onde d'électrocardiographie mémorisée dans ledit moyen de mémoire (2).

6. Appareil pour enregistrer un électrocardiogramme selon la revendication 5, dans lequel les points maximums négatifs détectés par lesdits moyens de détection (3) sont pris en tant que lesdits points de référence pour les groupes QRS respectifs.

7. Appareil pour enregistrer un électrocardiogramme selon l'une quelconque des revendications 1 à 4, dans lequel ledit point de référence dans chaque groupe QRS est situé à la position d'un pic d'une pointe dans ledit groupe QRS.

8. Appareil pour enregistrer un électrocardiogramme selon la revendication 7, dans lequel ladite pointe est une onde R du groupe QRS.

9. Appareil pour enregistrer un électrocardiogramme selon l'une quelconque des revendications précédentes, dans lequel le moyen de commande de visualisation (7) est adapté pour commander l'unité de visualisation (9) afin d'afficher trois groupes QRS simultanément, le point de référence du groupe QRS le plus à gauche parmi les trois groupes QRS affichés étant situé à ladite position prédéterminée.

10. Appareil pour enregistrer un électrocardiogramme selon la revendication 2 ou l'une quelconque de ses revendications dépendantes, dans lequel ledit moyen de commande de visualisation (7) est également adapté pour commander l'unité de visualisation (9) afin d'afficher lesdites fréquences instantanées de battement cardiaque.

11. Appareil pour enregistrer un électrocardiogramme selon l'une quelconque des revendications précédentes, dans lequel l'unité de visualisation (9) comprend un dispositif de visualisation à cristaux liquides.

12. Appareil selon l'une quelconque des revendications précédentes, dans lequel ledit moyen de commande est actionnable afin d'amener la forme d'onde d'électrocardiogramme affichée à se décaler le long dudit axe de temps d'une quantité correspondant à un nombre prédéterminé de groupes QRS.

13. Appareil selon la revendication 12, dans lequel ledit nombre prédéterminé est variable en accord avec l'actionnement de ladite unité d'entrée à touches par l'utilisateur.
